# EUROPEAN PATENT APPLICATION

(11) **EP 4 834 310 A1**
(43) Date of publication of application: **04.11.2026**
(21) Application number: 24912438.9
(22) Date of filing: 13.12.2024
(51) Int. Cl.: A61K 8/06, A61K 8/29, A61K 8/35, A61Q 17/04

(54) **WATER-IN-OIL EMULSION COMPOSITION**

(30) Priority: 28.12.2023 JP 2023222429
(71) Applicant: Shiseido Company, Ltd., Chuo-ku Tokyo 104-0061 (JP)
(72) Inventor: MIZUGUCHI, Akari, Tokyo 104-0061 (JP); KATORI, Takahiro, Tokyo 104-0061 (JP)
(74) Representative: Ter Meer und Partner mbB
(86) International application number: PCT/JP2024/044170
(87) International publication number: WO 2025/142549

(57) **Abstract**

A water-in-oil emulsion composition contains: (A) a dibenzoylmethane derivative; and (B) a metal oxide powder hydrophobically treated with a non-silicone-based substance, and is substantially free of an aluminum element.

## Description

### TECHNICAL FIELD

The present disclosure relates to a water-in-oil emulsion composition.

### BACKGROUND ART

As water-in-oil emulsion compositions for cosmetics and the like, those that have an ultraviolet protection ability have been known. Such compositions generally contain a metal oxide powder, whose surface is subjected to a hydrophobization treatment, as an ultraviolet scattering agent. As the hydrophobization treatment agent, one that contains a silicone-based substance is often used from the viewpoint of improving the dispersibility of the ultraviolet scattering agent and consequently enhancing the ultraviolet protection ability of the composition (for example, PTL 1).

However, in recent years, in consideration of the achievement of the Sustainable Development Goals, it is required to reduce or avoid use of silicone-based substances, which are pointed out to be environmentally hazardous. Use of non-silicone-based substances as hydrophobization treatment agents has been studied.

In addition, as for ultraviolet absorbers as well, various components that can improve the ultraviolet protection ability of water-in-oil emulsion compositions have been studied. A dibenzoylmethane derivative is known as an ultraviolet absorber having a high ultraviolet protection ability, especially a high ultraviolet protection ability at high wavelengths (for example, PTL 2).

### CITATION LIST

### PATENT LITERATURE

PTL 1: Japanese Patent Application Laid-Open Publication No. 2021-167286
PTL 2: Japanese Patent Application Laid-Open Publication No. 2014-139228

### SUMMARY OF THE INVENTION

### TECHNICAL PROBLEM

Considering the above background, a formulation in which a metal oxide powder treated with a non-silicone-based hydrophobization treatment agent, and a dibenzoylmethane derivative are combined is conceivable. However, it has been found that some combinations of a metal oxide powder treated with a non-silicone-based hydrophobization treatment agent and a dibenzoylmethane derivative cause discoloration. A composition that has discolored is disadvantageous when it is used by being applied to an application target surface, because the original color of the application target surface is adversely affected.

In view of the above, it is an object of one embodiment of the present disclosure to provide a water-in-oil emulsion composition having ultraviolet protection ability that is designed to reduce environmental impact and hardly discolors.

### SOLUTION TO THE PROBLEM

A water-in-oil emulsion composition contains: (A) a dibenzoylmethane derivative; and (B) a metal oxide powder hydrophobically treated with a non-silicone-based substance, and is substantially free of an aluminum element.

### ADVANTAGEOUS EFFECTS OF THE INVENTION

According to one embodiment of the present disclosure, it is possible to provide a water-in-oil emulsion composition having an ultraviolet protection ability that is designed to reduce environmental impact and hardly discolors.

### DETAILED DESCRIPTION OF THE INVENTION

### <Water-in-Oil Emulsion Composition>

One embodiment of the present disclosure is a water-in-oil (W/O) emulsion composition containing: (A) a dibenzoylmethane derivative; and (B) a metal oxide powder hydrophobically treated with a non-silicone-based substance, and is substantially free of an aluminum element.

The use of the water-in-oil composition according to the present embodiment is not particularly limited, yet it is preferable to use it in the fields of cosmetics, quasi-drugs, and personal care products. The water-in-oil composition according to the present embodiment is suitable as an external preparation or a skin liniment, particularly as a cosmetic. More specifically, the composition may be a sunblock (sunscreen) for the purpose of ultraviolet protection, or a cosmetic having an enhanced ultraviolet protection function; for example, basic cosmetics, such as a foundation and the like, makeup cosmetics, and the like. The water-in-oil emulsion composition according to the present embodiment may be in a solid or semisolid state. It may also be provided in a state called a gel state, a jelly state, or a cream state.

### <Ultraviolet Absorber >

### <<(A) Dibenzoylmethane Derivative>>

The dibenzoylmethane derivative is an ultraviolet absorber that exhibits a high ultraviolet protection ability, and that can provide an ultraviolet protection effect even in a small amount. The dibenzoylmethane derivative can exhibit a high ultraviolet absorption function especially in a long wavelength region, specifically in a wavelength range of 300 nm or longer and 400 nm or shorter.

Specific examples of dibenzoylmethane derivatives include t-butylmethoxydibenzoylmethane (i.e., 4-tert-butyl-4'-methoxydibenzoylmethane), 2-methyldibenzoylmethane, 4-methyldibenzoylmethane, 4-isopropyldibenzoylmethane, 4-tert-butyldibenzoylmethane, 2,4-dimethyldibenzoylmethane, 2,5-dimethyldibenzoylmethane, 4,4'-diisopropyldibenzoylmethane, 4,4'-dimethoxydibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane, 2,6-dimethyl-4-tert-butyl-4'-methoxydibenzoylmethane, 1-(4-tert-butylphenyl)-3-(2-hydroxyphenyl)propane-1,3-dione, 1-(4-methoxy-1-benzofuran-5-yl)-3-phenylpropane-1,3-dione, and the like. Among these, t-butylmethoxydibenzoylmethane is preferable because it can provide a particularly high ultraviolet protection ability even in a small amount.
(A) The content of the dibenzoylmethane derivative is preferably 0.1% by mass or greater and 10% by mass or less, more preferably 0.5% by mass or greater and 8% by mass or less, and even more preferably 1% by mass or greater and 5% by mass or less relative to the total amount of the water-in-oil emulsion composition. When the content of the dibenzoylmethane derivative is 0.1% by mass or greater, an ultraviolet protection ability, especially an ultraviolet protection ability at long wavelengths, can be improved. When the content of the dibenzoylmethane derivative is 10% by mass or less, an ultraviolet protection ability in a good balance over the entire ultraviolet wavelength range can be obtained. In addition, it is possible to avoid an excessive increase in the amount of a polar oil, and to avoid inhibiting the stability of the properties of the water-in-oil emulsion composition.

### <Ultraviolet Absorber Other Than Dibenzoylmethane Derivative>

The water-in-oil emulsion composition according to the present embodiment may contain an ultraviolet absorber other than the dibenzoylmethane derivative. Examples of the ultraviolet absorber other than the dibenzoylmethane derivative include β,β-diphenylacrylate derivatives, salicylic acid derivatives, cinnamic acid derivatives, benzoic acid derivatives, benzophenone derivatives, benzylidene camphor derivatives, phenylbenzimidazole derivatives, triazine derivatives, phenylbenzotriazole derivatives, anthranil derivatives, imidazoline derivatives, benzalmalonate derivatives, 4,4-diarylbutadiene derivatives, and the like.

Examples of the β,β-diphenylacrylate derivative include octocrylene and the like. Examples of the salicylic acid derivative include ethylhexyl salicylate (ethylhexylsalicylate or octyl salicylate), homosalate, dipropylene glycol salicylate, TEA salicylate, and the like. Among these, ethylhexyl salicylate is preferable. Examples of the cinnamic acid derivative include ethylhexyl methoxycinnamate (octyl methoxycinnamate), isopropyl methoxycinnamate, isoamyl methoxycinnamate, cinoxate, DEA methoxycinnamate, diisopropyl methylcinnamate, glyceryl-ethylhexanoate-dimethoxycinnamate, di-(2-ethylhexyl)-4'-methoxybenzalmalonate, and the like. Among these, ethylhexyl methoxycinnamate is preferable.

Examples of the benzoic acid derivative include ethyl p-aminobenzoate (PABA), ethyl-dihydroxypropyl PABA, ethylhexyl-dimethyl PABA, glyceryl PABA, PEG-25-PABA, hexyl diethylaminohydroxybenzoyl benzoate, and the like. Examples of the benzophenone derivative include benzophenone-1, benzophenone-2, benzophenone-3 or oxybenzone, benzophenone-4, benzophenone-5, benzophenone-6, benzophenone-8, benzophenone-9, benzophenone-12, and the like. Examples of the benzylidene camphor derivative include 3-benzylidene camphor, 4-methylbenzylidene camphor, benzylidene camphor sulfonic acid, camphor benzalkonium methosulfate, terephthalylidene dicamphor sulfonic acid, polyacrylamide methylbenzylidene camphor, and the like. Examples of the phenylbenzimidazole derivative include phenylbenzimidazole sulfonic acid, disodium phenyldibenzimidazole tetrasulfonate, and the like. Examples of the triazine derivative include anisotriazine(bisethylhexyloxyphenol methoxyphenyltriazine), ethylhexyltriazone, diethylhexyl butamido triazone, 2,4,6-tris(diisobutyl-4'-aminobenzalmalonate)-s-triazine, and the like. Examples of the phenylbenzotriazole derivative include drometrizole trisiloxane, methylenebis (benzotriazolyl tetramethylbutylphenol), and the like. Examples of the anthranil derivative include menthyl anthranilate and the like. Examples of the imidazoline derivative include ethylhexyl dimethoxybenzylidene dioxoimidazolidine propionate-2-ethylhexyl and the like. Examples of the benzalmalonate derivative include polyorganosiloxane having a benzalmalonate functional group, and the like. Examples of the 4,4-diarylbutadiene derivative include 1,1-dicarboxy(2,2'-dimethylpropyl)-4,4-diphenylbutadiene, and the like.

One of the above-mentioned ultraviolet absorbers other than the dibenzoylmethane derivative can be used alone, or two or more of them can be used in combination. A preferable combination of ultraviolet absorbers is a combination of the dibenzoylmethane derivative, and octocrylene and ethylhexyl salicylate, or a combination of the dibenzoylmethane derivative and ethylhexyl methoxycinnamate. This combination can widen the wavelength range in which the ultraviolet absorbers exhibit the ultraviolet absorption ability, as compared with the case where only the dibenzoylmethane derivative is used.

The content of the dibenzoylmethane derivative may be preferably 5% by mass or greater and 50% by mass or less and more preferably 10% by mass or greater and 40% by mass or less relative to the total amount of the ultraviolet absorber in the water-in-oil emulsion composition. In the above range, the ultraviolet absorption ability of the water-in-oil emulsion composition, especially in the long wavelength region, can be enhanced, and an ultraviolet absorption ability with a good balance over the entire ultraviolet wavelengths can be obtained.

### <(B) Metal Oxide Powder Hydrophobically Treated with Non-Silicone-Based Substance>

The water-in-oil emulsion composition according to the present embodiment further contains a hydrophobically treated metal oxide powder. The hydrophobically treated metal oxide powder is a metal oxide powder, the surface of which is treated with a hydrophobization treatment agent, and that has a hydrophobic surface. It is preferable that the hydrophobically treated metal oxide powder is one that has an ultraviolet protection ability, specifically, an ultraviolet scattering function, that is, it is preferable that the hydrophobically treated metal oxide powder is an ultraviolet scattering agent. Further, since a non-silicone-based substance (described later in detail) is used, the hydrophobically treated metal oxide powder is a metal oxide powder hydrophobically treated with (B) the non-silicone-based substance.

### <<Metal Oxide Powder>>

The hydrophobically treated metal oxide powder contained in the water-in-oil emulsion composition as an ultraviolet scattering agent may be in the form of particles. In this case, the average primary particle diameter of the metal oxide powder is not particularly limited, yet may be 5 nm or greater, 10 nm or greater, or 15 nm or greater, and may be 200 nm or less, 100 nm or less, or 50 nm or less. The "average primary particle diameter" in this specification may be obtained as, for example, an equivalent projected circle area diameter of a primary particle in an SEM image.

Specific examples of the metal oxide powder include titanium oxide, zinc oxide, cerium oxide, iron oxide, tungsten oxide, and the like. One or more of these metal oxide powders may be used alone, or two or more of these may be used in combination. Among these, titanium oxide and zinc oxide are preferable because of their high ultraviolet scattering function. Further, titanium oxide is preferable because of its high scattering function for ultraviolet rays in a relatively low wavelength region, especially ultraviolet rays of 280 nm or longer and 320 nm or shorter. Further, when titanium oxide is combined with the dibenzoylmethane derivative described above, it is possible to impart an ultraviolet protection ability in a wide wavelength region to the water-in-oil emulsion composition. Further, by using titanium oxide as the metal oxide powder, it is possible to improve the transparency of the water-in-oil emulsion composition to be obtained. The "transparency" of the water-in-oil emulsion composition refers to the transparency when applied to an application target surface. When the water-in-oil emulsion composition has a low transparency, it leaves a so-called white cast on the application target surface, to make the application target surface seem white. This is particularly disadvantageous when the application target surface is skin, and the user wants to avoid this phenomenon.

Further, since the water-in-oil emulsion composition is substantially free of zinc oxide, the transparency of the water-in-oil emulsion composition can be improved. In the present specification, when it is said that something "is substantially free of a predetermined component", it means that unintentional mixing of an aluminum element in a raw material obtaining step and/or a manufacturing step is allowed.

### <<Surface Treatment>>

The hydrophobically treated metal oxide powder has a hydrophobic surface due to the treatment with the hydrophobization treatment agent. More specifically, a layer of the hydrophobization treatment agent may be formed on the entirety or a part of the surface of particles of the metal oxide powder. The hydrophobically treated metal oxide powder is dispersed mainly in an oil phase of the water-in-oil emulsion composition.

The hydrophobization treatment of the metal oxide powder is a treatment for introducing a hydrophobic group to, or forming a coating layer of the hydrophobization treatment agent on the entirety or a part of, the surface of particles of the metal oxide powder. The hydrophobization treatment may be performed by a wet method using a solvent, a gas phase method, a mechanochemical method, and the like.

In the hydrophobization treatment, a non-silicone-based substance is used as the hydrophobization treatment agent. Here, the non-silicone-based substance is a substance other than a silicone-based substance. By using a non-silicone-based substance as the hydrophobization treatment agent, it is possible to reduce the content of silicone in the water-in-oil emulsion composition and obtain a composition with reduced environmental impact, thereby contributing to the achievement of the sustainable development goals. In this specification, the term "silicone-based substance" refers to a substance based on an organopolysiloxane, and the "silicone-based substance" encompasses a silicone oil, a silicone-based surfactant, a silicone rubber, a silicone resin, and the like.

Specific examples of the hydrophobization treatment agent that is a non-silicone-based substance include alkyl phosphate esters, higher fatty acids, higher alcohols, higher fatty acid esters, dextrin fatty acid esters, metal soaps, higher hydrocarbons, and the like. Among these, alkyl phosphate esters are preferable.

Monoalkyl phosphate is preferable as the alkyl phosphate ester. Further, as the alkyl phosphate ester, higher alkyl phosphate esters, that is, phosphate esters containing an alkyl group containing six or more carbon atoms are preferable, phosphate esters containing a linear or branched alkyl group containing eight or more and 30 or less carbon atoms are more preferable, and phosphate esters containing a linear alkyl group containing 10 or more and 20 or less carbon atoms are more preferable. The alkyl phosphate ester may be in the form of an ester or in the form of a salt thereof (alkyl phosphate salt or alkyl phosphate ester salt). Specific examples of the alkyl phosphate ester include cetyl phosphate (monocetyl phosphate), octyl phosphate, decyl phosphate (monodecyl phosphate), lauryl phosphate (dodecyl phosphate), myristyl phosphate, cetearyl phosphate, stearyl phosphate, and the like, among which cetyl phosphate is particularly preferable. Since the metal oxide powder treated with the hydrophobization treatment agent containing cetyl phosphate exhibits high dispersibility in the composition, it is possible to impart an ultraviolet protection ability comparable to that obtained by using a hydrophobization treatment agent composed of a silicone-based substance. Cetyl phosphate is also preferable from the viewpoint of promoting reduction of environmental impact.

Examples of the higher fatty acid include stearic acid, isostearic acid, myristic acid, and the like. Examples of dextrin fatty acid ester include dextrin palmitate and the like.

One of the hydrophobization treatment agents may be used alone, or two or more of them may be used in combination.

In addition to the hydrophobization treatment, a treatment with a hydrophilization treatment agent may be applied to the surface of the metal oxide powder. In this case, the hydrophilization treatment agent may be mixed with the hydrophobization treatment agent in advance and the surface treatment of the metal oxide powder may be performed using the mixture treatment agent. Further, the surface of the metal oxide powder may be subjected to a treatment using a hydrophilization treatment agent (hydrophilization treatment), and then subjected to a treatment using a hydrophobization treatment agent (hydrophobization treatment). In this specification, the hydrophobization treatment agent and the hydrophilization treatment agent may be collectively referred to as a surface treatment agent.

The hydrophilization treatment agent may also be a non-silicone-based substance. Specifically, the hydrophilization treatment agent may be an inorganic silicic acid compound, a metal hydroxide, and the like. Examples of the inorganic silicic acid compound include untreated silica. Examples of the metal oxide include magnesium hydroxide and the like. Among these, untreated silica is preferable as the hydrophilization treatment agent. When untreated silica is used, the particle surface of the metal oxide powder is more reliably coated, and metal ions of the metal oxide powder are less likely to precipitate. Further, since the dispersibility of the metal oxide powder can be further improved, a feeling that is felt during use of the water-in-oil emulsion composition can be improved.

One or more of the above hydrophilization treatment agents can be used alone, or two or more of them may be used in combination.

When the surface treatment agent contains a hydrophobization treatment agent and a hydrophilization treatment agent and an alkyl phosphate ester is used as the hydrophobization treatment agent, it is preferable to use untreated silica as the hydrophilization treatment agent. Furthermore, in this case, it is preferable to apply the hydrophilization treatment to the particle surface of the metal oxide powder to form a silica coating, and then apply the hydrophobization treatment to form an alkyl phosphate ester coating, to form a double coating on the particle surface of the metal oxide powder. By forming the silica coating in advance, it is possible to form the alkyl phosphate ester coating more uniformly and more reliably.

When the metal oxide powder hydrophobically treated with the non-silicone-based substance is contained as the ultraviolet scattering agent, the content of (B) the metal oxide powder hydrophobically treated with the non-silicone-based substance may be preferably 0.1% by mass or greater and 30% by mass or less, more preferably 1% by mass or greater and 25% by mass or less, even more preferably 3% by mass or greater and 20% by mass or less, and particularly preferably 4% by mass or greater and 13% by mass or less relative to the total amount of the water-in-oil emulsion composition. When the content of the metal oxide powder hydrophobically treated with the non-silicone-based substance as the ultraviolet scattering agent is 0.1% by mass or greater, the water-in-oil emulsion composition can exhibit a sufficient ultraviolet protection function. When the content is 30% by mass or less, the dispersion state of the ultraviolet scattering agent in the water-in-oil emulsion composition is improved, and the transparency of the water-in-oil emulsion composition is also improved.

### <Aluminum Element>

In the present embodiment, the water-in-oil emulsion composition is substantially free of an aluminum element. Here, the term "aluminum element" encompasses an aluminum simple substance and an aluminum ion. Further, when it is said that the "water-in-oil emulsion composition is substantially free of an aluminum element", it means that presence of the aluminum element in some form in the water-in-oil emulsion composition due to unintentional mixing of the aluminum element in a raw material obtaining step and/or a manufacturing step is allowed. When it is said that the "water-in-oil emulsion composition is substantially free of an aluminum element", it means that the content of an aluminum element (the content of an aluminum simple substance or an aluminum ion) is preferably 1% by mass or less, more preferably 0.5% by mass or less, and even more preferably 0.05% by mass or less relative to the total amount of the water-in-oil emulsion composition. Further, the content of an aluminum element is particularly preferably 0% by mass relative to the total amount of the water-in-oil emulsion composition.

As described above, the water-in-oil emulsion composition according to the present embodiment contains the dibenzoylmethane derivative as an ultraviolet absorber. Here, the present inventors have found that presence of an aluminum element, even in such an amount in which it is contained in a surface treatment agent of an ultraviolet scattering agent, causes discoloration that can affect the appearance of the water-in-oil emulsion composition as a final product. This is considered to be caused by formation of a complex between the dibenzoylmethane derivative and an aluminum ion in the composition, which causes the composition to develop a red color. When the water-in-oil emulsion composition is used as a skin liniment, such discoloration is undesirable for a user because the color of the skin itself appears discolored. In this regard, since the surface treatment agent for the metal oxide powder is substantially free of an aluminum element, discoloration (red discoloration) can be prevented.

Here, according to the present embodiment, since the water-in-oil emulsion composition is substantially free of an aluminum element, discoloration caused by the action between the dibenzoylmethane derivative and an aluminum element can be prevented.

Specifically, the surface treatment agent for the ultraviolet scattering agent is substantially free of an aluminum element. Further, an oil phase or a water phase of the water-in-oil emulsion composition is substantially free of an aluminum compound or the aluminum metal. The meaning of "substantially free of" is as described above.

The discoloration in the present specification may be evaluated immediately after the preparation of the final product (water-in-oil emulsion composition), yet, in particular, may be evaluated by observing the change in color after a predetermined time has elapsed. Further, it is preferable to perform evaluation by observing the change in color after a predetermined time has elapsed at a high temperature. "After the elapse of a predetermined time" may be when a time amounting to some days or longer and some weeks or shorter has elapsed after the preparation of the product. The "high temperature" may be a temperature exceeding the normal temperature (15°C to 25°C), and may be 30°C or higher, 40°C or higher, or 50°C or higher. The evaluation of the discoloration of the water-in-oil emulsion composition according to the present embodiment may be, for example, the evaluation of presence or absence of red discoloration in a state of being stored at 50°C for four weeks after the preparation.

### <Organically Modified Clay Mineral>

An organically modified clay mineral can function as an emulsification aid for assisting emulsification of an oil-based component and a water-based component. More specifically, by adding the organically modified clay mineral, it is possible to thicken or gel the oil-based component suitably, and to maintain the gelled state of the oil phase over time, which also makes it possible to stabilize the dispersed state of the water phase. Thus, the water-in-oil state can be stably maintained for a long time.

The organically modified clay mineral may be a cationmodified clay mineral obtained by treating a layered clay mineral, such as bentonite, laponite, hectorite, montmorillonite, and aluminum magnesium silicate, and the like with a quaternary ammonium salt-type cationic surfactant. Specific examples of the organically modified clay mineral include disteardimonium hectorite (dimethyldistearammonium hectorite), dimethylalkylammonium hectorite, benzyldimethylstearylammonium hectorite, magnesium aluminum silicate treated with distearyldimethylammonium chloride, and the like. Among these, it is preferable to use disteardimonium hectorite because of its high emulsifying function. The organically modified clay minerals described above can be formulated singly or in combination of two or more types.

The content of the organically modified clay mineral is preferably 0.05% by mass or greater and 10% by mass or less, and more preferably 0.1% by mass or greater and 5% by mass or less relative to the total amount of the water-in-oil emulsion composition. When the content of the organically modified clay mineral is 0.05% by mass or greater, the water-in-oil state can be stabilized. Since the ultraviolet scattering agent is suitably dispersed in the composition in the stable state, the ultraviolet protection ability is improved. When titanium oxide is used as the metal oxide powder, the ultraviolet protection ability in a low wavelength region can be improved. In addition, when the content of the organically modified clay mineral is 5% by mass or less, when, for example, the water-in-oil emulsion composition is a skin liniment, a pleasant, less tacky feel during use is obtained, and the composition spreads easily on the skin, and usability is improved.

### <Silicone-Based Substance>

As described above, no silicone-based substance is used as the hydrophobization treatment agent for the metal oxide powder hydrophobically treated with the non-silicone-based substance. In the present embodiment, it is further preferable that the water-in-oil composition as a whole is substantially free of a silicone-based substance. The term "silicone-based substance" refers to an organopolysiloxane-based substance, and encompasses silicone oil, silicone rubber, silicone-based active agent, silicone resin, and the like. When the water-in-oil composition as a whole is substantially free of a silicone-based substance, a silicone-free product or a silicone-reduced product can be provided, thereby reducing environmental impact and contributing to the realization of a sustainable society. The meaning of "substantially free of" is as described above. When it is said that "the water-in-oil emulsion composition is substantially free of a silicone-based substance", it means that the content of a silicone-based substance is preferably 1% by mass or less, more preferably 0.5% by mass or less, and even more preferably 0.05% by mass or less relative to the total amount of the water-in-oil emulsion composition. Furthermore, the content of a silicone-based substance is particularly preferably 0% by mass relative to the total amount of the water-in-oil emulsion composition.

In particular, it is preferable that the water-in-oil emulsion composition is substantially free of a silicone oil as an oily component. The meaning of "substantially free of" is as described above. When it is said that "the water-in-oil emulsion composition is substantially free of a silicone oil", it means that the content of a silicone oil is preferably 1% by mass or less, more preferably 0.5% by mass or less, and even more preferably 0.05% by mass or less relative to the total amount of the water-in-oil emulsion composition. Furthermore, the content of a silicone oil is particularly preferably 0% by mass relative to the total amount of the water-in-oil emulsion composition.

It is also preferable that a surfactant is substantially free of a silicone-based surfactant and that the ultraviolet absorber is substantially free of a silicone-based ultraviolet absorber, such as a polysilicone derivative and the like.

### <Other Components>

The water-in-oil emulsion composition according to the present embodiment may contain other optional components in addition to the above-mentioned components. Examples of the other components include water as the main component of a water phase, a water-soluble alcohol, an oily component other than the ultraviolet absorber, a surfactant (including a surfactant-like component), an inorganic powder other than the ultraviolet scattering agent, an antiseptic, a stabilizer, a pH adjuster, a thickener, a pigment, an antioxidant, an anti-inflammatory agent, a whitening agent, a plant extract, an activator, a blood circulation promoter, an antiseborrheic agent, and the like. When the water-in-oil emulsion composition is a skin liniment, components normally used in external compositions, such as cosmetics, quasi-drugs, and the like, may be used as the other components, and may be added in an amount that does not interfere with the effects according to the present embodiment.

### <<Main Component of Water Phase>>

As the water, purified water, ion-exchanged water, tap water, and the like can be used. Examples of the water-soluble alcohol include lower alcohols, polyhydric alcohols, polyhydric alcohol polymers, alcohol alkyl ethers, alcohol ether esters, glycerin monoalkyl ethers, sugar alcohols, derivatives thereof, and the like.

Examples of the lower alcohol include ethanol, propanol, isopropanol, isobutyl alcohol, t-butyl alcohol, and the like. Examples of the polyhydric alcohol include dihydric alcohols (for example, dipropylene glycol, 1,3-butylene glycol, ethylene glycol, trimethylene glycol, 1,2-butylene glycol, tetramethylene glycol, 2,3-butylene glycol, pentamethylene glycol, 2-butene-1,4-diol, hexylene glycol, octylene glycol, and the like), trihydric alcohols (for example, glycerin, trimethylolpropane, and the like), tetrahydric alcohols (for example, diglycerin, pentaerythritol (such as 1,2,6-hexanetriol and the like), and the like), pentahydric alcohols (xylitol, triglycerin, and the like), hexahydric alcohols (for example, sorbitol, mannitol, and the like), and the like. The polyhydric alcohol also functions as a moisturizer.

### <<Oily Component>>

Examples of the oily component include hydrocarbon oils, ester oils, higher fatty acids, higher alcohols, liquid oils and fats, solid oils and fats, waxes, and the like. Among these, hydrocarbon oils and ester oils are preferable, and it is more preferable to use a hydrocarbon oil and an ester oil in combination. The content of the oily component may be preferably 10% by mass or greater and 60% by mass or less, and more preferably 20% by mass or greater and 50% by mass or less relative to the total amount of the water-in-oil emulsion composition.

Examples of the hydrocarbon oil include isododecane, undecane, tridecane, isohexadecane, isoparaffin, liquid paraffin, ozokerite, squalane, pristane, paraffin, ceresin, squalene, Vaseline, microcrystalline wax, and the like. Among these, isododecane, undecane, tridecane, and squalane are preferable.

Examples of the ester oil include octyl palmitate, myristyl myristate, isononyl isononanoate, isopropyl myristate, cetyl 2-ethylhexanoate, octyldodecyl myristate, isopropyl palmitate, butyl stearate, hexyl laurate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di(2-ethylhexanoate), dipentaerythritol fatty acid ester, N-alkyl glycol monoisostearate, neopentyl glycol dicaprate, diisostearyl malate, glyceryl di(2-heptylundecanoate), trimethylolpropane tri(2-ethylhexanoate), trimethylolpropane triisostearate, pentaerythrit tetra 2-ethylhexanoate, glyceryl tri(2-ethylhexanoate) (triethylhexanoin), trimethylolpropane triisostearate, cetyl 2-ethylhexanoate, 2-ethylhexyl palmitate, diethylhexyl naphthalenedicarboxylate, alkyl benzoate (containing 12 to 15 carbon atoms), cetearyl isononanoate, glycerin tri(caprylate/caprate), butylene glycol (dicaprylate/caprate), glyceryl trimyristate, glyceride tri(2-heptylundecanoate), castor oil fatty acid methyl ester, oleyl oleate, cetostearyl alcohol acetoglyceride, 2-heptylundecyl palmitate, diisobutyl adipate, 2-octyldodecyl N-lauroyl-L-glutamate, di(2-heptylundecyl) adipate, ethyl laurate, di(2-ethylhexyl) sebacate, 2-hexyldecyl myristate, 2-hexyldecyl palmitate, 2-hexyldecyl adipate, diisopropyl sebacate, di(2-ethylhexyl succinate), ethyl acetate, butyl acetate, amyl acetate, triethyl citrate, 2-ethylhexyl p-methoxycinnamate, tripropylene glycol dipivalate, and the like. One of these ester oils may be used alone, or two or more of these ester oils may be used in combination.

Examples of the liquid oil and fat include jojoba seed oil, avocado oil, camellia oil, turtle oil, macadamia nut oil, corn oil, mink oil, olive oil, rapeseed oil, egg yolk oil, sesame oil, apricot kernel oil, wheat germ oil, sasanqua seed oil, castor oil, linseed oil, safflower oil, cottonseed oil, perilla oil, soybean oil, peanut oil, tea seed oil, Japanese Torreya oil, rice bran oil, China wood oil, Japanese tung oil, germ oil, triglycerin, and the like. Examples of the solid oil and fat include cacao butter, coconut oil, hydrogenated coconut oil, palm oil, horse fat, beef tallow, mutton tallow, hydrogenated beef tallow, palm kernel oil, pork fat, beef bone fat, Japan Tallow kernel oil, hydrogenated oil, neatsfoot fat, Japan Tallow, hydrogenated castor oil, and the like.

One or more of the above oily components may be used alone, or two or more of these may be used in combination.

### <<Surfactant>>

The surfactant (including a surfactant-like component) can function as an emulsifier and improve the stability of the emulsion system of the dosage form. The content of the surfactant may be preferably 0.5% by mass or greater and 8% by mass or less, and more preferably 1% by mass or greater and 5% by mass or less relative to the total amount of the water-in-oil emulsion composition. The surfactant (including a surfactant-like component) may be any of cationic, anionic, nonionic, or amphoteric surfactant. Among these, it is preferable to use a nonionic surfactant. Specific examples of the nonionic surfactant include: sorbitan fatty acid esters, such as sorbitan sesquiisostearate, sorbitan sesquioleate, sorbitan isostearate, sorbitan palmitate, and the like; polyethylene glycol fatty acid esters, such as PEG-8 diisostearate, PEG-10 diisostearate, and the like; polyglycerin fatty acid esters, such as polyglyceryl diisostearate-2 diisostearate, polyglyceryl-2 triisostearate, and the like; polyoxyethylene hydrogenated castor oil, such as PEG-40 hydrogenated castor oil, PEG-60 hydrogenated castor oil, PEG-100 hydrogenated castor oil, and the like; monoglycerol fatty acid esters, such as glyceryl stearate, glyceryl oleate, and the like; self-emulsifying propylene glycol stearate; and the like. Further, examples of the surfactant-like component include higher fatty acids, such as isostearic acid and the like. One of these surfactants can be used alone, or two or more of these can be used in combination.

### <Inorganic Powder>

The inorganic powder other than the ultraviolet scattering agent is an inorganic powder having no ultraviolet scattering function. Examples include silica, talc, kaolin, mica, sericite, muscovite, phlogopite, synthetic mica, lepidolite, biotite, vermiculite, magnesium carbonate, calcium carbonate, barium silicate, calcium silicate, magnesium silicate, strontium silicate, metal tungstate, magnesium, zeolite, barium sulfate, calcined calcium sulfate (calcined gypsum), calcium phosphate, fluorapatite, hydroxyapatite, ceramic powder, metal soap, boron nitride, and the like. Other examples include inorganic white pigments (for example, titanium dioxide, zinc oxide, and the like), inorganic red pigments (for example, iron oxide (red iron oxide), iron titanate, and the like), inorganic brown pigments (for example, γ-iron oxide, and the like), inorganic yellow pigments (for example, yellow iron oxide, ocher, and the like), inorganic black pigments (for example, black iron oxide, lower titanium oxide, and the like), inorganic purple pigments (for example, mango violet, cobalt violet, and the like), inorganic green pigments (for example, chromium oxide, chromium hydroxide, cobalt titanate, and the like), inorganic blue pigments (for example, ultramarine, navy blue, and the like), pearl pigments (for example, titanium oxide-coated mica, titanium oxide-coated bismuth oxychloride, titanium oxide-coated talc, colored titanium dioxide-coated mica, bismuth oxychloride, fish scale flake, and the like), metal powder pigments (for example, copper powder, and the like), organic pigments, such as zirconium lake, barium lake, and the like; natural pigments; and the like. These inorganic powders may be untreated, or subjected to surface treatment (hydrophobization treatment and/or hydrophilization treatment). One of these inorganic powders may be used alone, two or more of these may be used in combination.

The content of the inorganic powder other than the ultraviolet scattering agent may be preferably 1% by mass or greater and 30% by mass or less, and more preferably 5% by mass or greater and 20% by mass or less relative to the total amount of the water-in-oil emulsion composition.

### [Examples]

Hereinafter, Experimental Examples are shown. The components shown in Table 1 were mixed by a routine method, to prepare composition samples of Examples 1 to 3 and Comparative Examples 1 to 3, which were then evaluated. Table 1 also shows the evaluation results.

### <Protection Ability>

In this Example, the ultraviolet protection ability mainly in the short wavelength region was evaluated. A composition sample obtained in each example was dropped onto an S-plate (a 5 cm × 5 cm V-grooved PMMA plate, SPFMASTER-PA01) in an amount of 2 mg/cm², applied using a finger for 60 seconds, and dried for 15 minutes, and the absorbance (290 nm) of the composition sample was measured with a spectrophotometer (U-3500, obtained from Hitachi Ltd.). The absorbance (Abs) was calculated by the following equation, where T represents the transmittance through the composition sample, and To represents the transmittance through the plate to which the composition sample was not applied. $Abs = - log \left(T/To\right)$

The composition samples were evaluated based on the following evaluation criteria using Example 1 as a control.
A: The absorbance was equal to or higher than the absorbance of Example 1.
B: The absorbance was lower than the absorbance of Example 1, and the difference in absorbance from Example 1 was 0.15 or less.
C: The absorbance was lower than the absorbance of Example 1, and the difference in absorbance from Example 1 was greater than 0.15 and 0.19 or less.
D: The absorbance was lower than the absorbance of Example 1, and the difference in absorbance from Example 1 was greater than 0.19.

### <Transparency>

The composition sample of each example was filled in a plastic cell having an optical path length of 10 mm× an optical path width of 10 mm, and the transmittance of the wavelength region of 500 nm was measured using a spectrophotometer (U-4100, obtained from Hitachi Ltd.). The composition samples were evaluated based on the following evaluation criteria using Example 1 as a control.
A: The transmittance was higher than the transmittance of Example 1.
B: The transmittance was equal to or similar to the transmittance of Example 1.
C: The transmittance was lower than the transmittance of Example 1.

### <Discoloration>

The composition sample of each example was stored at 50°C for four weeks, and the color was visually observed and compared with the color before the storage. The discoloration was evaluated based on the following evaluation criteria.
A: No discoloration was observed.
B: Slight discoloration was observed.
C: Noticeable discoloration was observed.

**[Table 1]**

| **Component (% by mass)** | | **Ex. 1** | **Ex. 2** | **Ex. 3** | **Comp. Ex. 1** | **Comp. Ex. 2** | **Comp. Ex. 3** |
|---|---|---|---|---|---|---|---|
| **Water** | **Ion-exchanged water** | **Balance** | **Balance** | **Balance** | **Balance** | **Balance** | **Balance** |
| **Lower alcohol** | **Ethanol** | **6** | **6** | **6** | **6** | **6** | **6** |
| **Moisturizer** | **Glycerin** | **2** | **2** | **2** | **2** | **2** | **2** |
| | **Butylene glycol** | **1** | **1** | **1** | **1** | **1** | **1** |
| | **Dipropylene glycol** | **1** | **1** | **1** | **1** | **1** | **1** |
| **Organically modified clay mineral** | **Disteardimonium hectorite** | **0.3** | **0.3** | **0.3** | **0.3** | **0.3** | **0.3** |
| **Active agent** | **Isostearic acid** | **0.5** | **0.5** | **0.5** | **0.5** | **0.5** | **0.5** |
| | **Sorbitan sesquiisostearate** | **2** | **2** | **2** | **2** | **2** | **2** |
| | **PEG-8 diisostearate** | **0.5** | **0.5** | **0.5** | **0.5** | **0.5** | **0.5** |
| | **Polyglyceryl-2 diisostearate** | **4** | **4** | **4** | **4** | **4** | **4** |
| **Oily component** | **Isododecane** | **2** | **2** | **2** | **2** | **2** | **2** |
| | **Undecane** | **2** | **2** | **2** | **2** | **2** | **2** |
| | **Tridecane** | **2** | **2** | **2** | **2** | **2** | **2** |
| | **Squalane** | **15** | **15** | **15** | **15** | **15** | **15** |
| | **Jojoba seed oil** | **10** | **10** | **10** | **10** | **10** | **10** |
| | **Octyl palmitate** | **5** | **5** | **5** | **5** | **5** | **5** |
| | **Myristyl myristate** | **1** | **1** | **1** | **1** | **1** | **1** |
| **Ultraviolet absorber** | **Octocrylene** | **5** | **5** | **5** | **5** | **-** | **5** |
| | **Ethylhexyl salicylate** | **4.5** | **4.5** | **4.5** | **4.5** | **-** | **4.5** |
| | **t-butylmethoxydibenzoyl methane** | **2.5** | **2.5** | **2.5** | **-** | **-** | **2.5** |
| **Hydrophob. treated metal oxide powder (UV scattering agent)** | **Titanium oxide/silica/cetyl phosphate** | **5** | **15** | **-** | **5** | **-** | **-** |
| | **Titanium oxide/aluminum hydroxide/stearic acid** | **-** | **-** | **-** | **-** | **10** | **5** |
| | **Titanium oxide/magnesium hydroxide/stearic acid** | **-** | **-** | **5** | **-** | **-** | **-** |
| | **Zinc oxide/dextrin palmitate** | **-** | **-** | **-** | **10** | **10** | **-** |
| **Other additives** | **Silica** | **15** | **5** | **15** | **5** | **5** | **15** |
| | **Phenoxyethanol** | **0.5** | **0.5** | **0.5** | **0.5** | **0.5** | **0.5** |
| | **EDTA-3Na** | **0.2** | **0.2** | **0.2** | **0.2** | **0.2** | **0.2** |
| | **Table salt** | **0.1** | **0.1** | **0.1** | **0.1** | **0.1** | **0.1** |
| | **Sodium pyrosulfite** | **0.01** | **0.01** | **0.01** | **0.01** | **0.01** | **0.01** |
| | **Tocopherol** | **0.05** | **0.05** | **0.05** | **0.05** | **0.05** | **0.05** |
| | **Butyl hydroxy toluene (BHT)** | **0.05** | **0.05** | **0.05** | **0.05** | **0.05** | **0.05** |
| **Evaluation** | **Discoloration** | **A** | **A** | **A** | **A** | **A** | **C** |
| | **Protection ability *1** | **-** | **A** | **C** | **D** | **D** | **B** |
| | **Transparency *2** | **-** | **B** | **B** | **B** | **C** | **A** |

| | | | | | | | |
|---|---|---|---|---|---|---|---|
| *1, *2: Evaluation regarding Example 1 as control -PEG-8 diisostearate: EMALEX 400DI-IS EX, obtained from Nihon Emulsion Co., Ltd. -Bisbutyldimethicone polyglyceryl-3: Silicone KF-6109, obtained from Shin-Etsu Chemical Co., Ltd. -Polyglyceryl-2 diisostearate: WOGEL-18DV, obtained from Matsumoto Fine Chemical Co., Ltd. -Titanium oxide/silica/cetyl phosphate: Titanium oxide having an average primary particle diameter of 15 nm, whose particle surface was coated with silica and cetyl phosphate in this order -Titanium oxide/aluminum hydroxide/stearic acid: Titanium oxide having an average primary particle diameter of 15 nm, whose particle surface was coated with aluminum hydroxide and stearic acid in this order -Titanium oxide/magnesium hydroxide/stearic acid: Titanium oxide having an average primary particle diameter of 15 nm, whose particle surface was coated with magnesium hydroxide and stearic acid in this order -Zinc oxide/dextrin palmitate: Zinc oxide having an average primary particle diameter of 25 nm, whose particle surface was coated with dextrin palmitate | | | | | | | |

The percentages in Table 1 represent % by mass.

As shown in Table 1, it was found that the composition samples (Examples 1 to 3) that contain a dibenzoylmethane derivative and a metal oxide powder hydrophobically treated with a non-silicone-based substance and that were substantially free of an aluminum element did not cause discoloration and exhibited a high ultraviolet protection ability. It was also found that use of titanium oxide surface-treated with silica and cetyl phosphate as the metal oxide powder hydrophobically treated with a non-silicone-based substance (Examples 1 and 2) improved the ultraviolet protection ability and provided excellent transparency during application. Although the composition sample of Comparative Example 1 did not cause discoloration, its ultraviolet protection ability was low because it did not contain a dibenzoylmethane derivative.

Similarly, the composition samples of Examples 4 to 6 were also prepared and evaluated in terms of discoloration.

**[Table 2]**

| **Component (% by mass)** | | **Ex. 4** | **Ex. 5** | **Ex. 6** |
|---|---|---|---|---|
| **Water** | **Ion-exchanged water** | **Balance** | **Balance** | **Balance** |
| **Lower alcohol** | **Ethanol** | **6** | **6** | **6** |
| **Moisturizer** | **Glycerin** | **3** | **3** | **3** |
| | **Butylene glycol** | **1** | **1** | **1** |
| | **Dipropylene glycol** | **1** | **1** | **1** |
| **Organically modified clay mineral** | **Disteardimonium hectorite** | **0.3** | **0.15** | **1** |
| **Active agent** | **Isostearic acid** | **0.5** | **0.5** | **0.5** |
| | **Sorbitan sesquiisostearate** | **2** | **2** | **2** |
| | **PEG-8 diisostearate** | **0.5** | **0.5** | **0.5** |
| | **Polyglyceryl-2 diisostearate** | **4** | **4** | **4** |
| **Oily component** | **Isododecane** | **2** | **2** | **2** |
| | **Undecane** | **2** | **2** | **2** |
| | **Tridecane** | **2** | **2** | **2** |
| | **Squalane** | **15** | **15** | **15** |
| | **Jojoba seed oil** | **10** | **10** | **10** |
| | **Octyl palmitate** | **5** | **5** | **5** |
| | **Myristyl myristate** | **1** | **1** | **1** |
| **Ultraviolet absorber** | **Ethylhexyl methoxycinnamate** | **5** | **5** | **5** |
| | **t-butylmethoxydibenzoyl methane** | **2.5** | **2.5** | **2.5** |
| **Hydrophob. treated metal oxide powder (UV scattering agent)** | **Titanium oxide/silica/cetyl phosphate** | **5** | **5** | **5** |
| **Other additives** | **Silica** | **15** | **15** | **15** |
| | **Phenoxyethanol** | **0.5** | **0.5** | **0.5** |
| | **EDTA-3Na** | **0.2** | **0.2** | **0.2** |
| | **Table salt** | **0.1** | **0.1** | **0.1** |
| | **Sodium pyrosulfite** | **0.01** | **0.01** | **0.01** |
| | **Tocopherol** | **0.05** | **0.05** | **0.05** |
| | **Butyl hydroxy toluene (BHT)** | **0.05** | **0.05** | **0.05** |
| **Evaluation** | **Discoloration** | **A** | **A** | **A** |

The composition samples (Examples 4 to 6) that contain a dibenzoylmethane derivative and a metal oxide powder hydrophobically treated with a silicone-based substance, more specifically, cetyl phosphate, and that were substantially free of an aluminum element were shown to not cause discoloration.

Although the present disclosure has been described based on specific embodiments and Examples, these embodiments and Examples are provided by way of example only, and the present disclosure is not limited by the above embodiments and Examples. Various changes, modifications, substitutions, deletions, additions, combinations, and the like are applicable within the scope of the present disclosure.

The present application claims priority to Japanese Patent Application No. 2023-222429, filed on December 28, 2023, which is hereby incorporated in its entirety.

## Claims

1. A water-in-oil emulsion composition, comprising:
(A) a dibenzoylmethane derivative; and
(B) a metal oxide powder hydrophobically treated with a non-silicone-based substance,
wherein the water-in-oil emulsion composition is substantially free of an aluminum element.

2. The water-in-oil emulsion composition according to claim 1,
wherein (A) the dibenzoylmethane derivative contains t-butylmethoxydibenzoylmethane.

3. The water-in-oil emulsion composition according to claim 1,
wherein the non-silicone-based substance contains monoalkyl phosphate.

4. The water-in-oil emulsion composition according to claim 3,
wherein the non-silicone-based substance contains cetyl phosphate.

5. The water-in-oil emulsion composition according to claim 1 or 2,
wherein the metal oxide powder is a titanium oxide powder.

6. The water-in-oil emulsion composition according to claim 1 or 2,
wherein the water-in-oil emulsion composition is substantially free of a silicone oil.
